# EUROPÄISCHE PATENTSCHRIFT

(11) **EP 1 087 690 B1**
(45) Veröffentlichungstag und Bekanntmachung des Hinweises auf die Patenterteilung: **16.10.2002**
(21) Anmeldenummer: 00953099.9
(22) Anmeldetag: 26.07.2000
(51) Int. Cl.: A61B 1/00, A61B 19/00

(54) **BEFESTIGUNGSELEMENT FÜR EIN MEDIZINISCHES INSTRUMENT UND MEDIZINISCHES INSTRUMENT MIT DERARTIGEM BEFESTIGUNGSELEMENT**
FASTENING ELEMENT FOR A MEDICAL INSTRUMENT AND MEDICAL INSTRUMENT COMPRISING SAID FASTENING ELEMENT
ELEMENT DE FIXATION POUR INSTRUMENT MEDICAL ET INSTRUMENT MEDICAL COMPRENANT LEDIT ELEMENT

(30) Priorität: 26.07.1999 DE 19935012
(43) Veröffentlichungstag der Anmeldung: 04.04.2001
(73) Patentinhaber: Karl Storz GmbH & Co. KG, 78532 Tuttlingen (DE)
(72) Erfinder: STIHL, Ewald, D-78187 Geisingen (DE)
(74) Vertreter: Heuckeroth, Volker, Dr.
(86) Internationale Anmeldenummer: EP0007205
(87) Internationale Veröffentlichungsnummer: WO01006911

(56) Entgegenhaltungen:
- DE-A- 4 119 171
- DE-C- 4 137 426
- US-A- 4 744 361
- US-A- 5 713 869

## Beschreibung

Die Erfindung betrifft ein Befestigungselement zum Befestigen eines stabförmigen Elements an einem Schaft eines medizinischen Instruments, mit einer Lasche zum zumindest teilweise umfänglichen Umgreifen des stabförmigen Elements, wobei die Lasche in eine seitliche Öffnung eines Schaftteils einsetzbar ist, und mit einem Spannmechanismus, der bei Betätigung die Lasche in Richtung aus der Öffnung heraus anzieht, wodurch das stabförmige Element an dem Schaftteil festgeklemmt wird, wobei die Klemmkraft des Spannmechanismus zum Anklemmen des stabförmigen Elements bei der Betätigung des Spannmechanismus kontinuierlich einstellbar ist, indem der Spannmechanismus einen Schraubmechanismus aufweist, der ein um eine Längsachse des Befestigungselements drehbares Betätigungselement aufweist, dessen Drehbewegung in eine Bewegung der Lasche in Längsrichtung des Befestigungselements umgesetzt wird.

Die Erfindung betrifft ferner ein medizinisches Instrument, bei dem ein solches Befestigungselement verwendet wird.

Ein Befestigungselement und ein Instrument der eingangs genannten Art sind aus der US-A-4 744 361 bekannt.

Bei medizinischen Instrumenten besteht häufig das Bedürfnis, das vorliegende Instrument durch Verwendung mit einem zusätzlichen Instrument oder Instrumententeil, das üblicherweise im wesentlichen stabförmig, d.h. langerstreckt wie der Schaft des Instruments selbst ausgebildet ist, in seiner Funktion zu erweitern.

Unter einem stabförmigen Element im Sinne der vorliegenden Erfindung wird beispielsweise ein Spülschaft, ein Optikschaft oder ein Instrumentenschaft eines weiteren Hilfsinstruments, beispielsweise einer Schere, Zange oder einer Kanüle, ein Taststab, ein Elektrodenträger, oder dergleichen, verstanden, um nur einige Beispiele zu nennen. "Stabförmig" ist unabhängig von der Außenkontur und unabhängig davon zu verstehen, ob das Element hohl (rohrförmig) ist oder massiv.

Der Schaft des medizinischen Instruments kann im Rahmen der vorliegenden Erfindung ein Rohrschaft, bspw. eines Laryngoskops, sein. Damit das zusätzlich verwendete stabförmige Element kein Sichthindernis darstellt oder den Außendurchmesser des Rohrschafts des medizinischen Instruments vergrößert, ist es in einem solchen Fall wünschenswert, das stabförmige Element im Inneren des Rohrschafts befestigen zu können.

Der Schaft des medizinischen Instruments kann im Rahmen der vorliegenden Erfindung jedoch auch so ausgebildet sein, daß die Schaftwand nicht wie bei einem Rohrschaft umfänglich geschlossen, sondern seitlich offen ist.

Aus der US-A-5 713 869 ist ein Befestigungselement bekannt, das eine Lasche aufweist, die durch eine seitliche
Öffnung des Schafts von außen in das Innere des Schafts einsetzbar ist. Als Spannmechanismus zum Anziehen der Lasche in Richtung aus der Öffnung des Schafts heraus ist ein mit einem Nocken versehener umlegbarer Hebel vorgesehen, wobei durch Umlegen des Hebels aus einer Stellung in Längsrichtung des Befestigungselements in eine Stellung quer zur Längsrichtung des Befestigungselements der Nocken mit der Außenwand des Schafts in Anlage kommt und beim Abwälzen an der Außenwand des Schafts die Lasche nach außen zieht.

Der Spannmechanismus dieses bekannten Befestigungselements hat jedoch den Nachteil, daß der Spannmechanismus nicht variabel ist, so daß sich das bekannte Befestigungselement nur zum Anklemmen eines stabförmigen Elements mit einem definierten Durchmesser eignet. Wird ein zu dünnes stabförmiges Element verwendet, kann der umlegbare Hebel die Lasche nicht weit genug aus der Öffnung heraus anziehen, um das stabförmige Element festzuklemmen. Wird ein zu dickes stabförmiges Element verwendet, läßt sich der Hebel unter Umständen nicht umlegen und die Lasche kann somit nicht im gespannten Zustand fixiert werden. Darüber hinaus besteht die Gefahr, daß bei stabförmigen Elementen mit einem größeren Durchmesser die Klemmkraft beim Umlegen des Hebels übermäßig groß ist, so daß das stabförmige Element verbogen oder gequetscht werden kann. Insbesondere im Fall, daß es sich bei dem stabförmigen Element um eine Endoskopoptik handelt, besteht die Gefahr einer Beschädigung der Optik durch eine übermäßige Klemmkraft.

Aus der DE 41 37 426 ist ferner ein Befestigungselement bekannt, daß im Unterschied zu dem eingangs genannte Befestigungselement in Längsrichtung des Schafts des Instruments in eine proximale Öffnung des Schafts eingesetzt und dort mit einem Spannbacken in der Art einer Schraubzwinge angeklemmt wird. In dem Befestigungselement ist eine Aufnahme zum Halten eines stabförmigen Elements vorgesehen, die eine Blattfeder zum Festklemmen des stabförmigen Elements in der Aufnahme aufweist, was jedoch nur eine unzureichende Maßnahme zum Festklemmen des stabförmigen Elements am Schaft darstellt.

Die eingangs genannte US-A-4 744 361 offenbart ein Befestigungselement zum Anklemmen einer Elektrode an einem Resektoskop, und zwar an einem Schlitten eines Handgriffes, der mit dem Schaft der Resektoskops verbunden ist. Das Befestigungselement weist zum Festlegen des proximalen Endes der Elektrode eine Lasche auf, in die das proximale Ende der Elektrode einführbar ist. Die Lasche ist in eine seitliche Öffnung des Schlittens einsetzbar. Dieses bekannte Befestigungselement weist einen Spannmechanismus auf, der eine Kombination aus einem Schraubmechanismus und einem federbelasteten Drückmechanismus darstellt. Eine Feder spannt die Lasche dazu in Richtung aus der Öffnung hinaus vor. Das Einsetzen der Elektrode in das Befestigungselement erfolgt demnach durch Herunterdrücken eines Druckknopfes gegen die Kraft der Feder, und durch Loslassen des Druckknopfes klemmt die Lasche aufgrund der Federwirkung das proximale Ende fest, und zwar an einem in den Schlitten eingeschraubten Sockel des Befestigungselements. Um das Befestigungselement bezüglich verschiedener Elektrodendurchmesser variabel zu gestalten, ist die Lasche des Befestigungselements über einen Schraubmechanismus mittels einer Drehhülse, mit der die Lasche bzw. ihr oberer Halter in Gewindeeingriff steht, höhenverstellbar, wodurch die Feder des Druckmechanismus in ihrer Vorspannung verstellt werden kann. Auf diese Weise kann die Klemmkraft des Spannmechanismus kontinuierlich eingestellt werden.

Das Schaftteil des Schafts, in dem die Öffnung zum Einsetzen der Lasche ausgebildet ist, kann bei der vorliegenden Erfindung durch die Wand des Schafts selbst gebildet sein, oder das Schaftteil kann durch einen mit dem Schaft verbundenen Halter gebildet sein.

Der Erfindung liegt die Aufgabe zugrunde, ein Befestigungselement der eingangs genannten Art bereitzustellen, mit dem stabförmige Elemente unterschiedlicher Querschnittsabmessungen rasch und in möglichst leicht zu handhabender Weise am Schaftteil befestigt werden können.

Erfindungsgemäß wird diese Aufgabe dadurch gelöst, daß der Spannmechanismus weiterhin eine feststehende Hülse aufweist, die einen mit einer Durchtrittsöffnung für die Lasche versehenen Bodenabschnitt aufweist, dessen Außenseite an der Außenseite des Schaftteils in Anlage kommt und als Widerlager für die kontinuierlich einstellbare Klemmkraft dient.

Durch das erfindungsgemäße Befestigungselement wird demnach eine Art Klemmadapter bereitgestellt, mit dem ein stabförmiges Element an dem Schaftteil des medizinischen Instruments auf leicht zu handhabende Weise sicher angeklemmt werden kann. Die Lasche des Befestigungselements wird dazu durch die Öffnung in dem Schaftteil eingesetzt, wonach das stabförmige Element in die Lasche eingeschoben werden kann. Anschließend wird der Spannmechanismus betätigt, wodurch die Lasche kontinuierlich in Richtung aus der Öffnung des Schaftteils heraus angezogen wird, bis das stab- oder rohrförmige Element am Schaft festgeklemmt ist. Aufgrund des kontinuierlich wirkenden Spannmechanismus ist das Befestigungselement für unterschiedliche stabförmige Elemente mit unterschiedlichen Querschnittsabmessungen universell verwendbar. Das erfindungsgemäße Befestigungselement ermöglicht es nun, beispielsweise einen Spülschaft oder eine Optik an dem Schaft zu befestigen. Durch die Ausgestaltung des erfindungsgemäßen Befestigungselements wird eine hohe, jedoch vom Benutzer mit Handkraft einstellbare Klemmkraft zum Festklemmen des stabförmigen Elements erreicht, so daß unterschiedlich dimensionierte stabförmige Elemente mit stets etwa gleicher Klemmkraft am Schaftteil festgeklemmt werden können. Das stabförmige Element wird trotz einer hohen Klemmkraft nicht beschädigt und verformt sich nicht.

Der Spannmechanismus weist einen Schraubmechanismus auf, der ein um eine Längsachse des Befestigungselements drehbares Betätigungselement aufweist, dessen Drehbewegung in eine Bewegung der Lasche in Längsrichtung des Befestigungselements umgesetzt wird.

Ein Spannmechanismus, der zum Anziehen der Lasche einen Schraubmechanismus aufweist, hat den Vorteil, daß sich die Handkraft beim Betätigen des Spannmechanismus vom Benutzer gut dosieren läßt, weil der Benutzer die erreichte Klemmkraft durch die zunehmende Schwergängigkeit des drehbaren Betätigungselements leicht feststellen kann. Des weiteren läßt sich ein solcher Schraubmechanismus auf einfache Weise selbsthemmend ausgestalten, so daß das stabförmige Element nach Anziehen der Lasche sich nicht lockern kann. Der Bodenabschnitt der feststehenden Hülse bildet dabei vorteilhafterweise das Widerlager für die kontinuierlich einstellbare Klemmkraft.

In einer bevorzugten Ausgestaltung weist der Spannmechanismus einen in Längsrichtung des Befestigungselements beweglichen Halter für die Lasche auf, an dem die Lasche befestigt ist, und der über einen Gewindeeingriff mit dem drehbaren Betätigungselement in Verbindung steht.

Hierbei ist von Vorteil, daß ein konstruktiv wenig Teile erfordernder Schraubmechanismus zum Umsetzen der Drehbewegung des Betätigungselements in eine translatorische Bewegung der Lasche geschaffen wird, der darüber hinaus selbsthemmend ist.

In einer weiteren bevorzugten Ausgestaltung legt die Außenseite des Bodenabschnitts feststehenden Hülse sich im wesentlichen formschlüssig an die Kontur des Schaftteils an.

Hierbei ist von Vorteil, daß aufgrund des sich im wesentlichen formschlüssig an das Schaftteil anlegenden Widerlagers eine besonders hohe Klemmkraft zum Festklemmen des stabförmigen Elements an dem Schaftteil aufgebracht werden kann, ohne daß das Schaftteil durch Punktbelastungen oder Kanten des Befestigungselements beschädigt wird.

In einer weiteren bevorzugten Ausgestaltung ist das Betätigungselement als Drehkappe ausgebildet, die die feststehende Hülse umgibt, wobei die feststehende Hülse den als zweite Hülse ausgebildeten Halter umgibt, wobei die Drehkappe mit einem sich durch den Halter erstreckenden Drehzapfen drehfest verbunden ist, der mit dem Halter in Gewindeeingriff steht.

Diese Bauweise des erfindungsgemäßen Befestigungselements gewährleistet eine einwandfreie Funktion des Befestigungselements, eine leichte Bedienbarkeit und darüber hinaus eine kleinbauende Bauweise, die bei medizinischen Instrumenten erwünscht ist.

Weiterhin ist es bevorzugt, wenn die Lasche einen teilkreisförmig erweiterten, radial elastischen Halteabschnitt aufweist, in den das stabförmige Element einführbar ist, der einen Durchmesser aufweist, der größer ist als die Querabmessung der Öffnung des Schaftteils.

Diese Maßnahme hat den Vorteil, daß das Befestigungselement nach dem Einsetzen in die Öffnung des Schaftteils aufgrund des teilkreisförmig erweiterten, radial elastischen Halteabschnitts an dem Schaftteil bereits selbsthaltend vorfixiert ist, bevor das stabförmige Element in die Lasche eingeschoben und die Lasche angezogen wird. Das Befestigungselement kann somit am Schaftteil in der Art eines Clips in die Öffnung eingesetzt werden und ist dabei gegen ein Herausfallen gesichert, auch wenn das stabförmige Element nicht in die Lasche eingesetzt wurde. Die Bedienung des Befestigungselements und die Handhabung beim Befestigen des stabförmigen Elements ist dadurch wesentlich verbessert.

Weiterhin ist es bevorzugt, wenn die Lasche mit einer geringen Materialstärke, bevorzugt in Form eines Bandes, ausgebildet ist.

Hierbei ist von Vorteil, daß die Lasche kein raumergreifendes Hindernis darstellt, weil es das stab- oder rohrförmige Element radial im wesentlichen nicht überragt.

Weiterhin ist es bevorzugt, wenn sich die Lasche in Richtung der Längsrichtung des stabförmigen Elements über eine Teillänge desselben erstreckt.

Hierbei ist von Vorteil, daß bereits mit einem einzigen erfindungsgemäßen Befestigungselement das stabförmige Element an dem Schaft des Instruments befestigt werden kann, ohne daß das stabförmige Element pendelt oder verkippt. Eine Breite der Lasche von etwa 0,5 bis 1 cm ist dafür bereits ausreichend, ohne daß die Erfindung hierauf beschränkt ist.

In einer weiteren bevorzugten Ausgestaltung ist an der Lasche auf der dem stabförmigen Element zugewandten Seite der Öffnung eine Gegenplatte vorgesehen.

Das Vorsehen einer solchen Gegenplatte kann vorteilhafterweise vermeiden, daß das stabförmige Element, wenn dieses einen sehr kleinen Durchmesser aufweist, beim Anziehen des Befestigungselements teilweise mit seinem Außenumfang in die Öffnung gezogen wird. Die Gegenplatte ist bevorzugt so ausgebildet, daß sie die Öffnung verschließt und gegebenenfalls als formschlüssiges Gegenlager für das stabförmige Element wirkt.

Die Erfindung betrifft ferner ein medizinisches Instrument, mit einem Schaft, wobei ein Befestigungselement nach einem der vorhergehenden Ausgestaltungen zum Befestigen eines stabförmigen Elements an dem Schaft vorgesehen ist.

In einer bevorzugten Ausgestaltung des Instruments ist das Schaftteil durch die Wand des Schafts selbst gebildet.

Dies kann beispielsweise bei einem Rohrschaftinstrument mit einem Rohrschaft vorteilhaft sein, um das stabförmige Element im Innern des Rohrschafts mittels des Befestigungselements festzuklemmen, wobei in einem solchen Fall konstruktiv einfach lediglich eine Öffnung in der Wand des Rohrschafts vorgesehen werden muß.

Es ist jedoch auch bevorzugt, wenn das Schaftteil ein mit dem Schaft verbundener Halter ist.

Ein solcher Halter kann dabei fest mit dem Schaft des Instruments verbunden sein, oder er kann, wie in einer weiteren bevorzugten Ausgestaltung angegeben ist, an verschiedenen Stellen des Schafts befestigbar ausgebildet sein. Das Halteelement kann beispielsweise selbst als Klemmteil ausgebildet sein, mit einer Klemmvorrichtung, durch die das Halteelement an verschiedenen Stellen des Schafts angeklemmt werden kann.

Weiterhin ist es bevorzugt, wenn die Öffnung in dem Schaftteil als Langloch ausgebildet ist, dessen Längsachse sich in Längsrichtung des Schafts erstreckt.

Dies hat den Vorteil, daß das Befestigungselement an verschiedenen Positionen in Längsrichtung des Schafts des Instruments gesehen angebracht werden kann, so daß auch das stabförmige Element an verschiedenen Positionen des Instruments festgeklemmt werden kann.

Des weiteren ist es bevorzugt, wenn eine Mehrzahl von Öffnungen am Schaftteil verteilt vorhanden sind.

Auch hieran ist vorteilhaft, daß bei entsprechender Anordnung der Öffnungen das stabförmige Element an verschiedenen Positionen am Schaft befestigt werden kann.

In einer weiteren bevorzugten Ausgestaltung des Instruments weist das Schaftteil zumindest teilweise einen bezüglich der Längsrichtung des Schafts gekrümmten Abschnitt auf, wobei die Öffnung als Langloch ausgebildet ist und sich zumindest über den gekrümmten Abschnitt des Schaftteils erstreckt.

Hierbei ist von Vorteil, daß das stabförmige Element in verschiedenen Winkellagen bezüglich der Längsrichtung des Schafts an dem Schaft befestigt werden kann. Diese Ausgestaltung eignet sich insbesondere für den Fall, daß das stabförmige Element eine Beobachtungsoptik ist, wodurch aufgrund der verschiedenen möglichen Winkeleinstellungen zwischen der Optik und dem Schaft verschiedene Blickrichtungen, relativ zu der Längsrichtung des Schafts, realisiert werden können.

Weitere Vorteile ergeben sich aus der nachfolgenden Beschreibung und der beigefügten Zeichnung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden hiernach mit Bezug auf diese näher beschrieben. Es zeigen:
- Fig. 1: eine perspektivische Ansicht eines ersten Ausführungsbeispiels eines medizinischen Instruments mit einem Rohrschaft, in dem mittels des Befestigungselements ein stabförmiges Element befestigt ist;
- Fig. 2: eine Seitenansicht der Anordnung aus Befestigungselement, Rohrschaft und mittels des Befestigungselements daran fixiertem stabförmigen Element;
- Fig. 3: einen Schnitt entlang der Linie III-III in Fig. 2;
- Fig. 4: ein zweites Ausführungsbeispiel eines medizinischen Instruments in Seitenansicht, wobei das Instrument einen seitlich offenen Schaft aufweist, an dem mittels des Befestigungselements aus Fig. 1 bis 3 ein stabförmiges Element fixiert ist;
- Fig. 5: eine Draufsicht auf das Instrument in Fig. 4;
- Fig. 6: einen proximalen Ausschnitt des Instruments in Fig. 4 und 5, wobei das stabförmige Element in einer gegenüber Fig. 4 abweichenden Winkelstellung bezüglich der Längsrichtung des Schafts an diesem fixiert ist; und
- Fig. 7: eine Fig. 6 ähnliche Darstellung, wobei das stabförmige Element in einer noch anderen Winkelstellung bezüglich der Längsrichtung des Schafts an diesem fixiert ist.

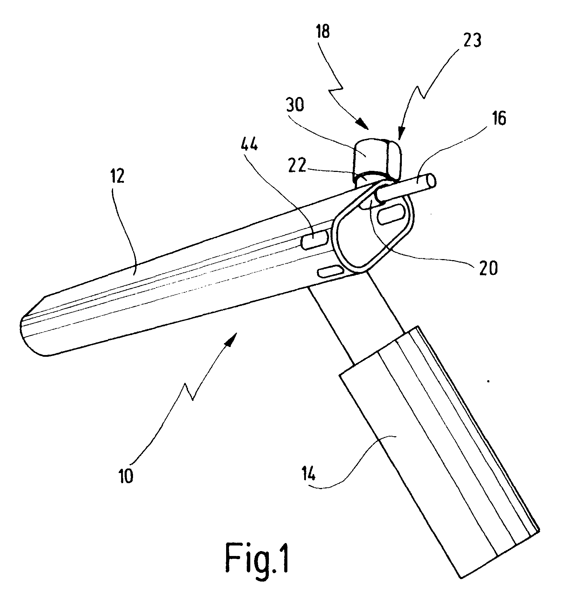

In Fig. 1 ist insgesamt ein Instrument 10 dargestellt, das einen Schaft 12 sowie einen daran befestigten Handgriff 14 aufweist. Der Schaft 12 ist als Rohrschaft ausgebildet. Das Instrument 10 ist hier ein Laryngoskop.

Im Inneren des Schafts 12 ist ein stabförmiges Element 16, das im gezeigten Ausführungsbeispiel ein Hohlschaft ist, dessen Außendurchmesser kleiner ist als der Innendurchmesser des Schafts 12, mittels eines erfindungsgemäßen Befestigungselements 18 an die Innenwand des Schafts 12 angeklemmt. Das stabförmige Element 16 ist hierbei ein Spülschaft, kann jedoch auch beispielsweise ein Optikschaft einer Endoskopoptik, oder ein sonstiges langerstrecktes Element von beliebiger Umfangskontur oder Querschnitt sein. Das Schaftteil, an dem das stabförmige Element 16 angeklemmt wird, ist bei diesem Ausführungsbeispiel somit der Schaft 12, genauer gesagt die Wand des Schafts 12, selbst.

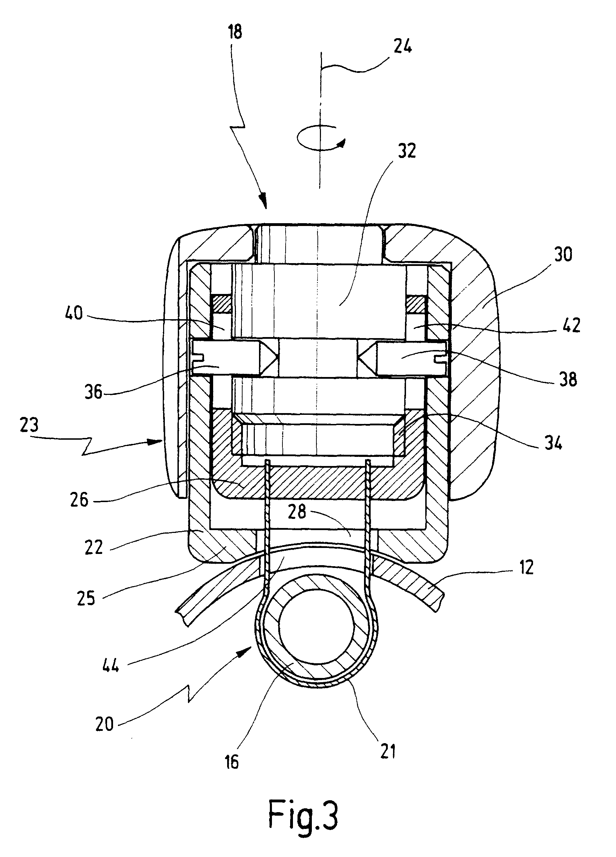

Mit Bezug auf Fig. 1 bis 3 wird nun das Befestigungselement 18, das auch als Klemmadapter bezeichnet werden kann, näher beschrieben.

Das Befestigungselement 18 weist zunächst eine Lasche 20 auf, die an ihrem unteren Ende einen im Querschnitt teilkreisförmig erweiterten, radial elastischen Halteabschnitt 21 aufweist.

Die Lasche 20 weist eine geringe Materialstärke auf. Die Lasche 20 kann beispielsweise durch ein flaches Metallband oder Kunststoffband gebildet sein.

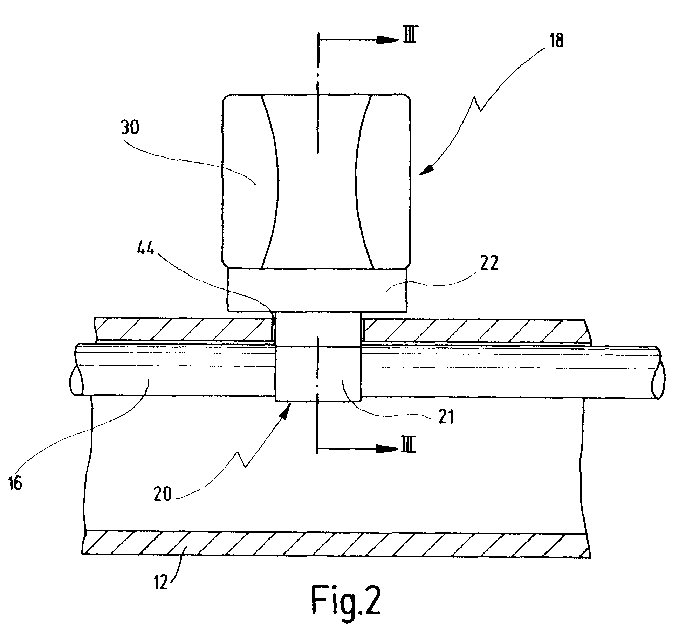

Die Lasche 20 weist weiterhin, wie aus Fig. 2 hervorgeht eine Ausdehnung in Richtung der Längsrichtung des stabförmigen Elements 16 auf, ist also in der Art eines breiten Bandes ausgebildet, beispielsweise mit einer Breite von 0,5 bis 1 cm.

Das Befestigungselement 18 weist ferner einen Spannmechanismus 23 auf, der bei Betätigung die Lasche 20, wie noch näher beschrieben wird, zum Festklemmen des stabförmigen Elements 16 an der Innenwand des Schafts 12 anzieht, und zwar kontinuierlich, so daß die Klemmkraft vom Benutzer mit Handkraft einstellbar ist, und dies unabhängig von der Querschnittsabmessung des stabförmigen Elements 16. Der Spannmechanismus 23 weist dabei einen Schraubmechanismus auf, der ein um eine Längsachse 24 des Befestigungselements 18 drehbares Betätigungselement aufweist, dessen Drehbewegung in eine translatorische Bewegung der Lasche 20 in Richtung der Längsachse 24 umgesetzt wird, wie hiernach noch näher beschrieben wird.

Das Befestigungselement 18 weist dazu eine erste feststehende Hülse 22 auf, d.h. die erste Hülse 22 ist bezüglich der Längsachse 24 weder axial verschiebbar noch um die Längsachse 24 verdrehbar. Die erste Hülse 22 weist einen Bodenabschnitt 25 auf, der im Gebrauch des Befestigungselements 18 an der Außenwand des Schafts 12 als Widerlager anliegt, und zwar formschlüssig.

Innerhalb der ersten feststehenden Hülse 22 ist eine zweite Hülse 26 angeordnet, an deren unterem Ende die Lasche 20 befestigt ist. Die Lasche 20 ist dazu durch eine Öffnung 28 im Bodenabschnitt 25 der ersten Hülse 22 durchgeführt.

Die zweite Hülse 26 ist bezüglich der Längsachse 24 unverdrehbar, jedoch axial in Richtung der Längsachse 24 beweglich. Aufgrund der Unverdrehbarkeit der zweiten Hülse 26 ist auch die Lasche 20 unverdrehbar.

Um die erste feststehende Hülse 22 herum ist als Betätigungselement des Spannmechanismus 23 eine Drehkappe 30 angeordnet, die um die Längsachse 24 drehbar ist. Die Drehkappe 30 ist fest mit einem inneren drehbaren Drehzapfen 32 verbunden, d.h. durch Drehen der Drehkappe 30 dreht sich der Drehzapfen 32 in der gleichen Richtung mit. Die Drehkappe 30 ist Bestandteil des Spannmechanismus 23 und dient als Betätigungselement zum Festziehen bzw. Spannen des Befestigungselements 18.

Der Drehzapfen 32 weist an seinem unteren Ende ein Außengewinde 34 auf, das mit einem entsprechenden Innengewinde in der zweiten Hülse 26 in Eingriff steht.

Die erste Hülse 22 ist über zwei Schrauben 36 und 38, die durch vertikale Langlöcher 40 und 42 hindurch geführt sind und in eine Ringnut in dem Zapfen 32 eingreifen, drehfest arretiert. Die vertikale Länge der Länglöcher 40 und 42 entspricht dem axialen Bewegungshub der zweiten Hülse 26.

Durch Drehen der Drehkappe 30 wird, wie bereits erwähnt der Drehzapfen 32 mitgedreht, und über den Gewindeeingriff zwischen dem Drehzapfen 32 und der zweiten Hülse 26 wird diese und mit dieser die Lasche 20 in Richtung der Längsachse 24 je nach Drehrichtung der Drehkappe 30 nach oben oder nach unten bewegt.

Die Funktion des Befestigungselements 18 zum Befestigen des stabförmigen Elements 16 an dem Schaft 12 ist wie folgt.

In dem Schaft 12 ist eine seitliche Öffnung 44 ausgespart. In diesem Fall ist die Öffnung 44 somit unmittelbar am Schaft 12 selbst ausgebildet, d.h. das Schaftteil wird durch einen Wandabschnitt des Schafts 12 selbst gebildet. Der teilkreisförmig erweiterte Halteabschnitt 21 der Lasche 20 weist dabei einen Durchmesser auf, der größer ist als die Breite der Öffnung 44, so daß beim Einstecken der Lasche 20 in die Öffnung 44 die Lasche 20 geringfügig elastisch zusammengedrückt wird und sich nach Passieren der Öffnung 36 wieder elastisch spreizt, wodurch das Befestigungselement 18 über den Halteabschnitt 21 der Lasche 20 in der Öffnung 44 in der Art einer Verrastung bzw. eines Clips gehalten ist.

In Fig. 1 sind in dem Schaft 12 vier derartige seitliche Öffnungen 44 vorgesehen, so daß das Befestigungselement 18 wahlweise in eine der Öffnungen 44 eingesetzt werden kann. Es ist somit möglich, das stabförmige Element 16 an verschiedenen Umfangspositionen an dem Schaft 12 anzuklemmen. Es können auch mehrere Befestigungselemente 18 vorgesehen sein, um entsprechend mehrere stabförmige Elemente 16 an dem Schaft 12 befestigen zu können. Es können auch in Längsrichtung des Schafts 12 verteilt mehrere solcher Öffnungen 44 vorgesehen sein, oder die Öffnung 44 ist als Langloch mit entsprechender Längsausdehnung ausgebildet, dessen Längsachse sich in Längsrichtung des Schafts 12 erstreckt.

Das Befestigungselement 18 wird nach oder vor dem Einstecken in die Öffnung 44 durch Drehen der Drehkappe 30 in seine Offenlage gebracht, in der die Lasche 20 entsprechend dem Durchmesser des stabförmigen Elements 16 weit genug aus der feststehenden Hülse 22 nach unten herausragt.

Anschließend wird das stabförmige Element 16 in den teilkreisförmigen Abschnitt der Lasche 20 eingeschoben. Durch Drehen der Drehkappe 30 in die entgegengesetzte Richtung um die Längsachse 24 wird dann die Lasche 20 in die feststehende Hülse 22 hineingezogen, d.h. in Richtung aus der Öffnung 44 des Schafts 12 heraus, wodurch das stabförmige Element 16 gegen die Innenwand des Schafts 12 gezogen und zwischen dem Halteabschnitt 21 und der Innenwand festgeklemmt wird, während der Bodenabschnitt 25 der feststehenden Hülse 22, der auf seiner Unterseite eine Wölbung aufweist, ein Widerlager gegen die von der Lasche 20 aufgebrachte Zugkraft bildet.

Durch Drehen der Drehkappe 30 kann somit die Lasche 20 soweit angezogen werden, bis das stabförmige Element 16 fest an dem Rohrschaft 12 angeklemmt ist.

Durch die axiale Ausdehnung der Lasche 20 in Längsrichtung des Rohrschafts 12 wird der Innenschaft 16 pendelfrei und ohne zu verkippen an dem Schaft 12 angeklemmt.

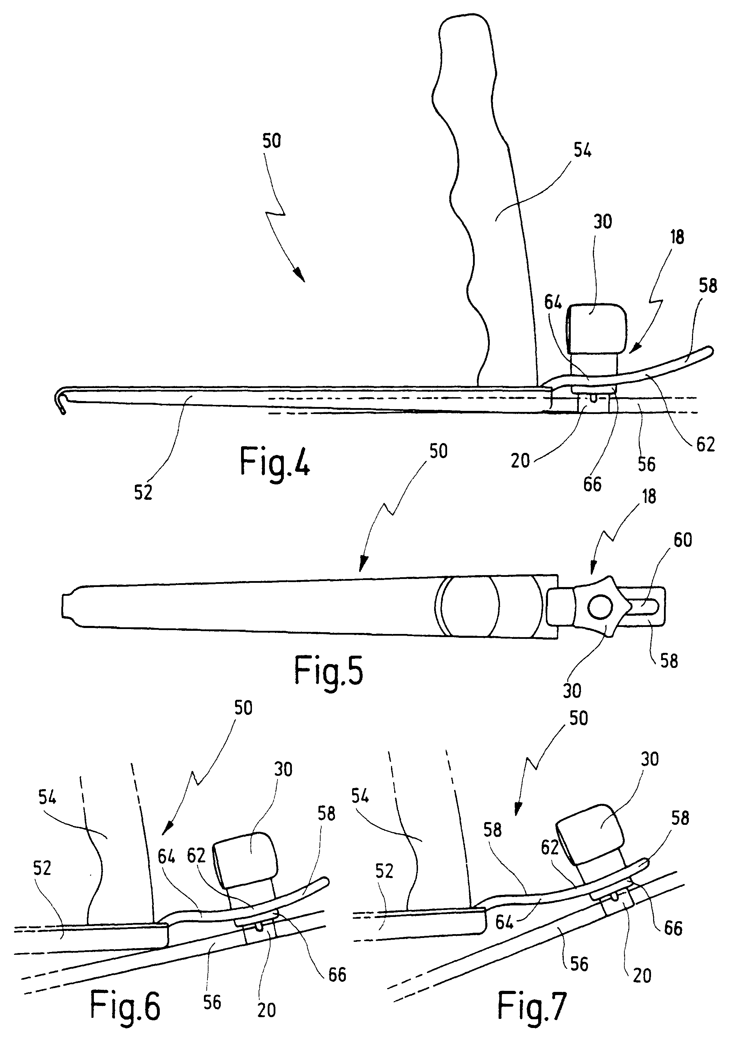

In Fig. 4 bis 7 ist ein weiteres Ausführungsbeispiel eines Instruments 50 dargestellt, das einen Schaft 52 und einen Handgriff 54 aufweist.

Der Schaft 52 des Instruments 50 ist im Unterschied zu dem Schaft 12 des Instruments 10 nicht als Rohrschaft, sondern als seitlich offener Schaft ausgebildet.

Das zuvor im Zusammenhang mit Fig. 1 bis 3 beschriebene Befestigungselement 18 kann auch bei dem Instrument 50 dazu verwendet werden, ein stabförmiges Element 56 an dem Schaft 52 zu befestigen.

Die Befestigungsstelle befindet sich bei dem Instrument 50 jedoch nicht am Schaft 52 selbst, sondern an einem Schaftteil, das als Halter 58 am proximalen Ende des Schafts 52 mit diesem verbunden ist.

In dem Halter 58 ist eine Öffnung 60 ausgespart, in die die Lasche 20 des Befestigungselements 18 einsteckbar ist, wie in Fig. 4 bis 7 dargestellt ist.

Die Öffnung 60 ist als Langloch ausgebildet, das sich im wesentlichen über die gesamte Länge des Halters 58 erstreckt.

Der Halter 58 weist weiterhin einen bezüglich der Längsrichtung des Schafts 52 gekrümmten Abschnitt 62 und einen etwa parallel zur Längsrichtung des Schafts verlaufenden geraden Abschnitt 64 auf.

Aufgrund der Ausgestaltung des Halter 58 mit einem gekrümmten Abschnitt 62 und einem geraden Abschnitt 64 ist es möglich, durch entsprechende Positionierung des Befestigungselements 18 an einer bestimmten axialen Stelle des Halters 58 das stabförmige Element 56 an dem Schaft 52 so zu befestigen, daß das stabförmige Element 56 parallel zur Längsrichtung des Schafts 52 verläuft (Fig. 4), oder bezüglich der Längsrichtung des Schafts 52 unter verschiedenen Winkelstellungen, wie in Fig. 6 und 7 dargestellt ist.

Bei dem in Fig. 4 bis 7 gezeigten Ausführungsbeispiel weist das Befestigungselement 18 weiterhin noch eine Gegenplatte 66 auf, die auf der dem stabförmigen Element 56 zugewandten Seite der Öffnung 60 auf die Lasche 20 aufgesteckt ist. Durch die Gegenplatte 66, die die Öffnung 60 von der dem stabförmigen Element 56 zugewandten Seite her verschließt, wird für das stabförmige Element 56 ein definiertes Widerlager gebildet, was insbesondere bei einer Ausgestaltung des Halteelements 58 mit einem gekrümmten Abschnitt 62 von Vorteil ist.

Während der Halter 58 am proximalen Ende des Schafts 52 in dem gezeigten Ausführungsbeispiel angeordnet ist, ist es ebenso denkbar, den Halter 58 an einer anderen Position des Schafts 52 anzuordnen.

Des weiteren ist es möglich, den Halter 58 abnehmbar von dem Schaft 52 auszugestalten und mit einer Befestigungseinrichtung auszustatten, beispielsweise mit einer Klemmvorrichtung, die es erlaubt, den Halter 58 an einer beliebigen Stelle am Schaft 52 abnehmbar zu befestigen.

Ein Langloch mit einem gekrümmten Verlauf wie die Öffnung 60 kann außerdem beispielsweise direkt in die Wand des Schafts 52 integriert werden oder auch bei dem Schaft 12 des Instruments 10 vorgesehen werden.

## Patentansprüche

1. Befestigungselement zum Befestigen eines stabförmigen Elements (16; 56) an einem Schaft (12; 52) eines medizinischen Instruments (10; 50), mit einer Lasche (20) zum zumindest teilweise umfänglichen Umgreifen des stabförmigen Elements (16; 56), wobei die Lasche (20) in eine seitliche Öffnung (44; 60) eines Schaftteils einsetzbar ist, und mit einem Spannmechanismus (23), der bei Betätigung die Lasche (20) in Richtung aus der Öffnung (44; 60) heraus anzieht, wodurch das stabförmige Element (16; 56) an dem Schaftteil (12) festgeklemmt wird, wobei die Klemmkraft des Spannmechanismus (23) zum Anklemmen des stabförmigen Elements (16; 56) bei der Betätigung des Spannmechanismus (23) kontinuierlich einstellbar ist, indem der Spannmechanismus (23) einen Schraubmechanismus aufweist, der ein um eine Längsachse des Befestigungselements drehbares Betätigungselement (30) aufweist, dessen Drehbewegung in eine Bewegung der Lasche in Längsrichtung des Befestigungselements umgesetzt wird, **dadurch gekennzeichnet, daß** der Spannmechanismus (23) weiterhin eine feststehende Hülse (22) aufweist, die einen mit einer Durchtrittsöffnung (28) für die Lasche (20) versehenen Bodenabschnitt (25) aufweist, dessen Außenseite an der Außenseite des Schaftteils in Anlage kommt und als Widerlager für die kontinuierlich einstellbare Klemmkraft dient.

2. Befestigungselement nach Anspruch 1, **dadurch gekennzeichnet, daß** der Spannmechanismus (23) einen in Längsrichtung des Befestigungselements beweglichen Halter für die Lasche (20) aufweist, an dem die Lasche (20) befestigt ist, und der über einen Gewindeeingriff mit dem Betätigungselement in Verbindung steht.

3. Befestigungselement nach Anspruch 1 oder 2, **dadurch gekennzeichnet, daß** die Außenseite des Bodenabschnitts (25) der feststehenden Hülse (22) sich im wesentlichen formschlüssig an die Kontur des Schaftteils anlegt.

4. Befestigungselement nach Anspruch 2 oder 3, soweit dieser auf Anspruch 2 rückbezogen ist, **dadurch gekennzeichnet, daß** das Betätigungselement als Drehkappe (30) ausgebildet ist, die die feststehende Hülse (22) umgibt, wobei die feststehende Hülse (22) den als zweite Hülse (26) ausgebildeten Halter umgibt, wobei die Drehkappe (30) mit einem sich durch den Halter erstreckenden Drehzapfen (32) drehfest verbunden ist, der mit dem Halter im Gewindeeingriff steht.

5. Befestigungselement nach einem der Ansprüche 1 bis 4, **dadurch gekennzeichnet, daß** die Lasche (20) einen teilkreisförmig erweiterten, radial elastischen Halteabschnitt (21) aufweist, in den das stabförmige Element (16; 56) einführbar ist, der einen Durchmesser aufweist, der größer ist als die Querabmessung der Öffnung (44; 60) des Schaftteils.

6. Befestigungselement nach einem der Ansprüche 1 bis 5, **dadurch gekennzeichnet, daß** die Lasche (20) mit einer geringen Materialstärke, bevorzugt in Form eines Bandes, ausgebildet ist.

7. Befestigungselement nach einem der Ansprüche 1 bis 6, **dadurch gekennzeichnet, daß** sich die Lasche (20) in Richtung der Längsrichtung des stabförmigen Elements (16; 56) über eine Teillänge desselben erstreckt.

8. Befestigungselement nach einem der Ansprüche 1 bis 7, **dadurch gekennzeichnet, daß** an der Lasche (20) auf der dem stabförmigen Element (56) zugewandten Seite der Öffnung (60) eine Gegenplatte (66) vorgesehen ist.

9. Medizinisches Instrument, mit einem Schaft (12; 52), **gekennzeichnet durch** ein Befestigungselement (18) nach einem der Ansprüche 1 bis 8 zum Befestigen eines stabförmigen Elements (16; 56) an dem Schaft (12; 52).

10. Instrument nach Anspruch 9, **dadurch gekennzeichnet, daß** das Schaftteil durch die Wand des Schafts (12) selbst gebildet ist.

11. Instrument nach Anspruch 9, **dadurch gekennzeichnet, daß** das Schaftteil ein mit dem Schaft (52) verbundener Halter (58) ist.

12. Instrument nach Anspruch 11, **dadurch gekennzeichnet, daß** der Halter (58) vom Schaft (52) abnehmbar ist.

13. Instrument nach Anspruch 11 oder 12, **dadurch gekennzeichnet, daß** der Halter (58) an verschiedenen Stellen des Schafts (52) befestigbar ist.

14. Instrument nach einem der Ansprüche 9 bis 13, **dadurch gekennzeichnet, daß** die Öffnung (44; 60) als Langloch ausgebildet ist, dessen Längsachse sich in Längsrichtung des Schafts (12; 53) erstreckt.

15. Instrument nach einem der Ansprüche 9 bis 14, **dadurch gekennzeichnet, daß** eine Mehrzahl von Öffnungen (44; 60) am Schaftteil verteilt ausgebildet sind.

16. Instrument nach einem der Ansprüche 9 bis 15, **dadurch gekennzeichnet, daß** das Schaftteil zumindest teilweise einen bezüglich der Längsrichtung des Schafts (52) gekrümmten Abschnitt (62) aufweist, und wobei die Öffnung (60) als Langloch ausgebildet ist und sich zumindest über den gekrümmten Abschnitt (62) des Schaftteils erstreckt.

## Claims

1. A fastening element for securing a rod-shaped element (16; 56) on a shaft (12; 52) of a medical instrument (10; 50), comprising a bracket (20) for engaging at least partially the circumference of the rod-shaped element (16; 56), the bracket (20) being insertable in a side opening (44; 60) of a portion of the shaft, and a tensioning mechanism (23) which when actuated draws the bracket (20) in the direction out of the opening (44; 60), whereby the rod-shaped element (16; 56) is clamped onto the shaft portion (12), wherein the clamping force of the tensioning mechanism (23) for clamping the rod-shaped element (16; 56) is continuously adjustable when actuating the tensioning mechanism (23) by that the tensioning mechanism (23) comprises a screw mechanism having an actuator element (30) rotatable about a longitudinal axis of the fastening element, whose rotary motion is converted into a motion of the bracket in the longitudinal direction of the fastening element, **characterized in that** the tensioning mechanism (23) further comprises a stationary bushing (22) having a base portion (25) provided with a through opening (28) for the bracket (20), an outer surface of the base portion (25) comes into engagement with the outer surface of the shaft portion and serves as an abutment for the continuously adjustable clamping force.

2. The fastening element of claim 1, **characterized in that** the tensioning mechanism (23) comprises a retainer for the bracket (20) which is moveable in the longitudinal direction of the fastening element, and to which the bracket (20) is secured and which is connected to the actuator element by engagement of a threading.

3. The fastening element of claim 1 or 2, **characterized in that** the outer surface of the base portion (25) of the stationary bushing (22) abuts on the contour of the shaft in substantially form-locking manner.

4. The fastening element of claim 2 or 3, as far as the latter is referenced back to claim 2, **characterized in that** the actuator element is configured as a turning cap (30) enclosing the stationary bushing (22), the stationary bushing (22) surrounding the retainer configured as a second bushing (26), wherein the turning cap (30) is fixidly connected to a pivot pin (32) extending through the retainer, which engages with the retainer through a threading.

5. The fastening element of any one of claims 1 through 4, **characterized in that** the bracket (20) comprises a radially elastic retainer section (21) formed to be partially circularly expanded into which the rod-shaped element (16; 36) can be inserted, the retainer section (21) having a diameter larger than the transverse dimension of the opening (44; 60) of the shaft portion.

6. The fastening element of any one of claims 1 through 5, **characterized in that** the bracket (20) has a low material thickness preferably being formed as a band.

7. The fastening element of any one of claims 1 through 6, **characterized in that** the bracket (20) extends over a partial length in the longitudinal direction of the rod-shaped element (16; 56).

8. The fastening element of any one of claims 1 through 7, **characterized in that** a counterplate (66) is provided on the bracket (20) at the side of the opening (60) facing the rod-shaped element (56).

9. A medical instrument comprising a shaft (12; 52), **characterized by** a fastening element (18) according to any one of claims 1 through 8 for securing a rod-shaped element (16; 56) to the shaft (12; 52).

10. The instrument of claim 9, **characterized in that** the shaft portion is formed by the wall of the shaft (12) itself.

11. The instrument of claim 9, **characterized in that** the shaft portion is a holder (58) connected to the shaft (52).

12. The instrument of claim 11, **characterized in that** the holder (58) is releasable from the shaft (52).

13. The instrument of claim 11 or 12, **characterized in that** the holder (58) can be secured at various positions on the shaft (52).

14. The instrument of any one of claims 9 through 13, **characterized in that** the opening (44; 60) is formed as a slotted hole whose longitudinal axis extends in the longitudinal direction of the shaft (12; 52).

15. The instrument of any one of claims 9 through 14, **characterized in that** a plurality of openings (44; 60) are formed to be distributed on the shaft portion.

16. The instrument of any one of claims 9 through 15, **characterized in that** the shaft portion comprises at least a portion (62) being cured with respect to the longitudinal direction of the shaft (52) and wherein the opening (60) is formed as a slotted hole which at least extends over the curved section (62) of the shaft portion.

## Revendications

1. Elément de fixation pour fixer un élément (16 ; 56) en forme de barre sur une tige (12 ; 52) d'un instrument médical (10 ; 50), comportant une attache (20) pour entourer périphériquement au moins en partie l'élément (16 ; 56) en forme de barre, l'attache (20) pouvant être insérée dans une ouverture latérale (44 ; 60) d'un élément formant tige, et comportant un mécanisme de tension (23) qui, lorsqu'il est actionné, tire l'attache (20) vers l'extérieur de l'ouverture (44 ; 60), ce qui fait que l'élément (16 ; 56) en forme de barre est serré contre l'élément formant tige (12), la force de serrage du mécanisme de tension (23) étant réglable en continu pour serrer l'élément (16 ; 56) en forme de barre pendant l'actionnement du mécanisme de tension (23), par le fait que le mécanisme de tension (23) comporte un mécanisme à vis qui comprend un élément d'actionnement (30) pouvant tourner autour d'un axe longitudinal de l'élément de fixation et dont le mouvement de rotation est converti en un mouvement de l'attache dans la direction longitudinale de l'élément de fixation, **caractérisé en ce que** le mécanisme de tension (23) comporte en outre une douille (22) fixe qui comprend une portion de fond (25) pourvue d'une ouverture de passage (28) pour l'attache (20) et dont le côté extérieur vient s'appliquer contre le côté extérieur de l'élément formant tige et sert de contre-butée à la force de serrage réglable en continu.

2. Elément de fixation selon la revendication 1, **caractérisé en ce que** le mécanisme de tension (23) comporte un support pour l'attache (20), lequel est déplaçable dans la direction longitudinale de l'élément de fixation et auquel est fixée l'attache (20), et qui est en liaison avec l'élément d'actionnement, par une liaison filetée.

3. Elément de fixation selon la revendication 1 ou 2, **caractérisé en ce que** le côté extérieur de la portion de fond (25) de la douille fixe (22) s'applique sensiblement par complémentarité de formes contre le contour de l'élément formant tige.

4. Elément de fixation selon la revendication 2 ou 3, dans la mesure où celle-ci se rattache à la revendication 2, **caractérisé en ce que** l'élément d'actionnement est conformé en capuchon tournant (30) qui entoure la douille fixe (22), la douille fixe (22) entourant le support conformé en deuxième douille (26), tandis que le capuchon tournant (30) est lié solidairement en rotation à un doigt tournant (32) qui s'étend à travers le support et qui est en liaison filetée avec ce dernier.

5. Elément de fixation selon l'une des revendications 1 à 4, **caractérisé en ce que** l'attache (20) comporte une portion de maintien (21) radialement élastique, élargie en forme de cercle partiel, dans laquelle peut être introduit l'élément (16 ; 56) en forme de barre qui présente un diamètre supérieur à la dimension transversale de l'ouverture (44 ; 60) de l'élément formant tige.

6. Elément de fixation selon l'une des revendications 1 à 5, **caractérisé en ce que** l'attache (20) est réalisée avec une faible épaisseur de matière, de préférence sous la forme d'un ruban.

7. Elément de fixation selon l'une des revendications 1 à 6, **caractérisé en ce que** l'attache (20) s'étend dans la direction longitudinale de l'élément (16 ; 56) en forme de barre, sur une partie de sa longueur.

8. Elément de fixation selon l'une des revendications 1 à 7, **caractérisé en ce qu'**il est prévu une contre-plaque (66) sur l'attache (20), sur le côté de l'ouverture (60) tourné vers l'élément (56) en forme de barre.

9. Instrument médical comportant une tige (12 ; 52), **caractérisé par** un élément de fixation (18) selon l'une des revendications 1 à 8, pour la fixation d'un élément (16 ; 56) en forme de barre sur la tige (12 ; 52).

10. Instrument selon la revendication 9, **caractérisé en ce que** l'élément formant tige est formé par la paroi de la tige (12) elle-même.

11. Instrument selon la revendication 9 **caractérisé en ce que** l'élément formant tige est un support (58) relié à la tige (52).

12. Instrument selon la revendication 11, **caractérisé en ce que** le support (58) peut être enlevé de la tige (52).

13. Instrument selon la revendication 11 ou 12, **caractérisé en ce que** le support (58) peut être fixé en différents emplacements de la tige (52).

14. Instrument selon la revendication 9 à 13, **caractérisé en ce que** l'ouverture (44 ; 60) est conformée en trou oblong dont l'axe longitudinal s'étend dans la direction longitudinale de la tige (12 ; 53).

15. Instrument selon l'une des revendications 9 à 14, **caractérisé en ce qu'**une pluralité d'ouvertures (44 ; 60) sont formées, réparties sur l'élément formant tige.

16. Instrument selon l'une des revendications 9 à 15, **caractérisé en ce que** l'élément formant tige comporte au moins en partie une portion (62) courbée par rapport à l'axe longitudinal de la tige (52), l'ouverture (60) étant conformée en trou oblong et s'étendant au moins sur la portion courbée (62) de l'élément formant tige.
